# EUROPÄISCHE PATENTANMELDUNG

(11) **EP 1 182 181 A1**
(43) Veröffentlichungstag der Anmeldung: **27.02.2002**
(21) Anmeldenummer: 01119686.2
(22) Anmeldetag: 23.08.2001
(51) Int. Cl.: C07C 2/78

(54) **Vormisch-Brennerblock für partielle Oxidationsprozesse**

(30) Priorität: 25.08.2000 DE 10041739
(71) Anmelder: BASF AKTIENGESELLSCHAFT, 67056 Ludwigshafen (DE)
(72) Erfinder: Bartenbach, Bernd, Dr., 67117 Limburgerhof (DE); Stapf, Dieter, Dr., 68199 Mannheim (DE); Bachtler, Michael, Dr., 76857 Albersweiler (DE); Scheidsteger, Olaf, Dr., 68163 Mannheim (DE); Pässler, Peter, Dr., 67069 Ludwigshafen (DE)
(74) Vertreter: Isenbruck, Günter, Dr.

(57) **Zusammenfassung**

Die vorliegende Anmeldung bezieht sich auf einen Brennerblock (1) enthaltend eine Basis (2), mehrere Kohlenwasserstoff-Kanäle (3) zum Durchtritt von als Reaktanden verwendeten Kohlenwasserstoff/Luft-Gemischen und mindestens einem Sauerstoffkanal (4) zum Durchtritt von Luft oder eines Luft/Sauerstoff-Gemischs. Optionsweise ist ein Zündbrenner (5) vorhanden. Der Brennerblock gestattet die Verbrennung von Kohlenwasserstoffen zur Herstellung von Acetylen bei Temperaturen < 1400°C.

## Beschreibung

Die vorliegende Erfindung betrifft einen neuartigen Brennerblock, der in partiellen Oxidationsprozessen, vorzugsweise der kombinierten Acetylen/Synthesegas-Herstellung aus Methan, Flüssiggas, Naphtha o.ä. Ausgangssubstanzen eingesetzt werden kann. Der Brennerblock eignet sich insbesondere für den Einsatz in partiellen Oxidationsprozessen, die in neu entwickelten Niedertemperatur-Fahrweisen durchgeführt werden.

Zahlreiche Verfahren zur unkatalysierten Herstellung von Acetylen basieren auf der Pyrolyse oder der partiellen Oxidation von Kohlenwasserstoffen. Es kommen dabei mit Erdgas, verschiedenen Erdöl-Fraktionen (z.B. auch Naphtha) und sogar Rückstandsölen (Tauchflammverfahren) eine breite Palette von Ausgangssubstanzen zum Einsatz. Prinzipiell haben bei pyrolytischen oder oxidativen Herstellungsverfahren von Acetylen thermodynamische und kinetische Parameter einen entscheidenden Einfluß auf die Wahl der Reaktionsbedingungen. Wichtige Voraussetzungen entsprechender Prozesse sind bei klassischen Verfahren eine schnelle Energiezufuhr bei einer Reaktionstemperatur von mehr als 1400°C, äußerst kurze Verweilzeiten der Einsatzstoffe bzw. Reaktionsprodukte von 10⁻² bis 10⁻³ Sekunden, niedriger Partialdruck des Acetylens und schnelles Abschrecken der entstandenen Gase. Acetylen fällt bei der Pyrolyse und bei der partiellen Oxidation in einem Gasgemisch, dem sogenannten Spaltgas an. Dabei enthält das Spaltgas normalerweise etwa 5 bis 20 Vol.-% Acetylen. Durch beispielsweise N-Methylpyrrolidon, Dimethylformamid, Kerosin, Methanol oder Aceton wird das Acetylen aus dem Spaltgas extrahiert und anschließend weiter gereinigt.

Die einzelnen Verfahren, bei denen Acetylen hergestellt wird, unterscheiden sich insbesondere hinsichtlich der Erzeugung der hohen Reaktionsemperaturen. Dabei spielen die Bereitstellung sowie die Übertragung der Wärmeenergie die entscheidende Rolle.

Das Sachsse-Bartholomé-Verfahren der Anmelderin ist ein sogenanntes autothermes Spaltverfahren und für Einsatzprodukte wie Methan, Flüssiggas oder Leichtbenzin geeignet. Die überwiegende Zahl der weltweit gebauten Anlagen basiert auf Erdgas als Einsatzprodukt; nur wenige verwenden Naphtha als Rohstoff. Im technischen Prozeß werden beispielsweise Methan und Sauerstoff getrennt auf 500 bis 600°C vorgeheizt, gemischt und in einem speziellen Brenner unter Flammenbildung zur Reaktion gebracht. Das Verhältnis O₂/CH₄ wird mit etwa 1:2 so eingestellt, daß nur eine unvollständige Verbrennung erfolgen kann. In der Flamme findet sowohl die exotherme Oxidation eines Teils des CH₄ als auch die endotherme Dehydrodimerisierung des CH₄ in Acetylen und Wasserstoff statt. Nach einer Verweilzeit von wenigen Millisekunden schreckt man das Reaktionsgas durch Einspritzen von Wasser oder Quenchöl ab. Dabei fallen pro 100 kg Acetylen etwa 5 kg Ruß an. Die Abtrennung des Acetylens wird überlicherweise mit einem Extraktionsmittel, wie N-Methylpyrrolidon oder Dimethylformamid, vorgenommen. Im Spaltgas beträgt der Volumenanteil des Acetylens etwa 8 %, die Hauptkomponenten sind Wasserstoff mit 57 Vol.-% und Kohlenmonoxid mit 26 Vol.-%, die in diesem Verhältnis ein gut einsetzbares Synthesegas darstellen.

Die Temperaturen bei diesem Verfahren liegen, wie auch bei den sonstigen bekannten Acetylen-Herstellungsmethoden, bei Werten von über 1400°C. Zur Vermeidung von Folgereaktionen liegen die Verweilzeiten im Millisekundenbereich, das Reaktionsgas muß dann schnell durch direktes Einspritzen eines Quenchmittels abgeschreckt werden. Dabei kühlt sich das entsprechende Gemisch je nach eingesetztem Quenchmittel stark ab, aus dem resultierenden Gemisch wird das Acetylen durch selektive Lösungsmittel ausgewaschen.

Eine Weiterentwicklung des vorstehend beschriebenen Prinzips der Acetylen/Synthesegas-Herstellung ist in der DE-A 44 22 815 der Anmelderin offenbart. Dabei wird das Ausgangsgemisch auf übliche Weise im Anschluß an die getrennte Vorwärmung in einer Mischkammer erzeugt. Die Verbrennung mit Acetylenbildung findet anschließend an einem in einem Brennraum angeordneten Brennerblock statt. Die Kanäle des Brennerblocks sind auf der Austrittseite mit einer Perforationen aufweisenden Platte abgedeckt. Dadurch kann in den gewonnenen Acetylen/Synthesegas-Gemisch der relative Anteil zwischen diesen beiden Komponenten in weiten Grenzen variiert werden.

Ein Nachteil des Sachsse-Bartholome-Verfahrens liegt darin, daß zum Erzielen einer hohen Acetylen-Konzentration im Spaltgas die als Ausgangsstoff eingesetzten Kohlenwasserstoffe und der Sauerstoff auf Temperaturen zwischen ca. 300 und 700°C vorgewärmt und dann vermischt werden. Bei einem Verhältnis Sauerstoff/Kohlenwasserstoff von ca. 1:2 wird so ein zündfähiges Gemisch erzeugt. Nur durch besondere konstruktive Maßnahmen, mit denen die Verweilzeit in der vor dem Brennerblock liegenden Mischzone kurz gehalten wird, läßt sich ein vorzeitiges Verbrennen des Kohlenwasserstoffs vermeiden. In der Handhabung eines zündfähigen Gemischs liegt jedoch immer ein gewisses Unsicherheits- und Gefahrenpotential. Bei Über- oder Unterschreiten gewisser Grenzwerte hinsichtlich der Prozeßparameter, aber auch durch vom Anlagenmaterial stammende Störungen, wie Eindringen von Rostpartikeln in das Gasgemisch, können nicht vorhergesehene Zündungen und Ausbeuteerniedrigungen eintreten.

Ein weiterer Nachteil liegt darin, daß durch das Quenchen eine beträchtliche Menge an Wärmemenge verloren geht bzw. eine optimale Rückgewinnung der Energie unmöglich wird. Die entsprechende Quenchflüssigkeit weist nach ihrem Einsatz typischerweise Temperaturen von höchstens 300°C auf, obwohl sie bei hohem Temperaturniveau von ca. 1500 bis 1600°C anfällt. Der Einsatzbereich einer solchen relativ kühlen Flüssigkeit zu Heizzwecken ist stark eingeschränkt. Der hohe energetische Aufwand, der für die Erzeugung der höher als 1400°C liegenden Reaktionstemperatur notwendig ist, ist schließlich ebenso als weiterer Nachteil zu nennen wie die starke Rußbildung, die bei den hohen Prozeßtemperaturen besonders ausgeprägt ist. Die Rußbildung reduziert nicht nur die Ausbeute an Synthesegas und Acetylen, aufwendig ist auch die Reinigung der durch Ruß bzw. Rußkoks verschmutzten Quenchflüssigkeit und des Spaltgasgemisches.

Um die vorstehend geschilderten Nachteile zu umgehen, wird in der DE-A 199 14 226.2 der Anmelderin ein Niedertemperatur-Verfahren offenbart, das durch einen prinzipiell anderen Verfahrensaufbau als bisher bekannt charakterisiert ist und zur Herstellung von Acetylen/Synthesegas-Gemischen durch partielle Verbrennung von Kohlenwasserstoffen geeignet ist. Dieses Verfahren zur Herstellung von Acetylen und Synthesegas durch thermische Behandlung eines geeigneten Kohlenwasserstoffs in Gegenwart von Sauerstoff wird im Unterschied zu den bislang bekannten Verfahren bei Temperaturen von maximal 1400°C durchgeführt.

Das Ausgangsgemisch zur Herstellung von Acetylen und Synthesegas kann beispielsweise durch Zünden, durch die Zufuhr von Energie oder durch exotherme Reaktionen bei gleichzeitiger oder vorausgehender Zufuhr von Energie aufgeheizt werden. Das Ausgangsgemisch bzw. aus dem Ausgangsgemisch entstehende Reaktionsmischungen weisen demnach erfindungsgemäß während des Verfahrens Temperaturen von maximal 1400°C auf.

Im Gegensatz zu bekannten Verfahren ist die mittlere Verweilzeit in dem Reaktor vergleichsweise lang und beträgt in der Regel mindestens 10 ms, in einer bevorzugten Ausführungsform wird durch indirekte Kühlung abgekühlt.

Die thermische Behandlung findet in der Regel bei Temperaturen zwischen 1200°C und 1400°C statt. Besonders bevorzugt ist dabei der Bereich bis 1350°C.

Der Vorteil der niedrigeren Prozeßtemperatur liegt zum einen in der geringen Rußbildung. Zum anderen ist der Energiebedarf deutlich niedriger als bei den bisherigen Verfahren, und eine effektive Rückgewinnung der Energie ist möglich.

Sauerstoff wird dem Ausgangsgemisch meist in Form von Luft, Sauerstoff oder Luft/Sauerstoff-Mischungen bereitgestellt. Möglich ist auch die Zugabe von Wasserdampf und/oder Kohlendioxid als Sauerstoffquelle. Bevorzugt ist die Zugabe von Sauerstoff oder Luft/Sauerstoff-Mischungen. Diese werden dem Reaktionsgemisch in einer solchen Menge zugegeben, daß das molare Verhältnis Sauerstoff/Kohlenstoff bei Werten von 0,1 bis 0,8, vorzugsweise 0,4 bis 0,6 liegt.

Die Zusammensetzung des Ausgangsgemisches richtet sich nach der Verwendug des herzustellenden Spaltgases. Wichtige Spaltgase, die mit dem dargestellten Verfahren hergestellt werden können, sind beispielsweise Acetylen/Methanol-Synthesegas, Acetylen/Ammoniaksynthesegas, Acetylen/Wasserstoff-Reichgas, Acetylen/Kohlenmonoxid-Reichgas, Acetylen/Oxogas, Acetylen/Ethylen-Synthesegas. Neben Erdgas können Flüssiggas (Propan, Butan), Leichtbenzin, Aromaten, Pyrolysebenzin-Öl (aus Crackprozessen) und/oder Vakuumdestillationsrückstände aus Erdölraffinerien verwendet werden. Ausgangsgemische können bis zu etwa 10 Vol.-% rückgeführtes Spaltgas, Recyclegase wie Restmethan aus der Spaltgasreinigung, Restgase aus anderen Prozessen oder Synthesegas beinhalten. Sauerstoff kann dem Ausgangsgemisch in Form von Luft bereitgestellt werden, wobei man Ammoniak-Synthesegas und Acetylen erhält, Wasserdampfzugabe zu Erdgas mit hohem Methananteil führt bevorzugt zu Acetylen/Wasserstoff-Reichgas und die Verwendung höherer Kohlenwasserstoffe ergibt ein Acetylen/Kohlenmonoxid-Reichgas.

Der Inhalt der Anmeldung DE-A 199 14 226.2 betreffend das Verfahren zur Herstellung von Acetylen/Synthsegasgemischen durch Verbrennen von Kohlenwasserstoffen in Gegenwart von Sauerstoff und/oder sauerstoffhaltigen Verbindungen ist ein wichtiger und integraler Bestandteil der vorliegenden Anmeldung und durch Referenz in die vorliegende Anmeldung eingeschlossen.

Das in der DE-A 199 14 226.2 offenbarte Verfahren, das in Abhängigkeit von der gewünschten Zusammensetzung des Acetylen/Synthesegas-Gemischs mit verschiedenen Zusammensetzungen der Ausgangsprodukte und bei verschiedenen Werten von Druck, Temperatur und Verweilzeit durchgeführt wird, sollte auch wünschenswerterweise so durchgeführt werden können, daß das Vorliegen von zündfähigen Gemischen der eingesetzten Reaktanden nicht mehr auftritt, bevor das Reaktandengemisch an dem Brenner unter Flammenentwicklung oxidiert wird.

Die Aufgabe der vorliegenden Erfindung liegt darin, einen Brennerblock zur Verfügung zu stellen, mit dem das Niedertemperaturverfahren zur Herstellung von Acetylen/Synthesgas-Gemischen ausgeführt werden kann und das Vorliegen dieser zündfähigen Gemische in diesem Verfahren vermieden werden kann.

Diese Aufgabe wird gelöst durch einen Brennerblock, der eine Basis aufweist, in der mehrere Kanäle angebracht sind, die zum Durchtritt von Kohlenwasserstoff/Sauerstoff-Gemischen bestimmt sind. Weiterhin ist ein Sauerstoffkanal vorhanden, durch den Luft, Sauerstoff oder ein Luft/Sauerstoff-Gemisch austritt.

Die Verbrennung des resultierenden Kohlenwasserstoff/Sauerstoff-Gemischs tritt nach dem Durchströmen des Brennerblocks ein.

Sauerstoff wird dem System in Form von Luft, Sauerstoff oder Sauerstoff/Luft-Gemischen zugeführt, möglich ist auch die Zugabe von Wasserdampf oder Kohlendioxid als Sauerstoffquelle. Dies gilt sowohl für das Kohlenwasserstoff/Sauerstoff-Gemisch als auch für den durch den Sauerstoffkanal eintretenden Sauerstoff. Alle die vorstehend genannten, als Sauerstoffquelle geeigneten Verbindungen werden nachfolgend als Sauerstoff bezeichnet.

Der erfindungsgemäße Brennerblock besteht aus einer Basis, in die mehrere Kohlenwasserstoff-Kanäle eingelassen sind. Durch diese tritt der als Ausgangsmaterial verwendete Kohlenwasserstoff, der etwa Methan, Erdgas, Flüssiggas (Propan/Butan), Leichtbenzin, ein Aromatengemisch, Pyrolysebenzin oder sogar Rückstandsöl sein kann, hindurch. Der Ausgangsstoff wurde zuvor mit Luft, Sauerstoff, einem Luft/Sauerstoff-Gemisch oder einer sauerstoffhaltigen Verbindung, etwa Wasser oder Kohlendioxid, vermischt. Der erfindungsgemäße Brennerblock erlaubt es dabei im Gegensatz beispielsweise zum Sachsse-Bartholome-Verfahren, dem Kohlenwasserstoff zunächst eine Menge an Sauerstoff zuzusetzen, mit der kein zündfähiges Gemisch gebildet und trotzdem eine stabile Zündung und Verbrennung im anschließenden Reaktionsraum erreicht wird. Das Kohlenwasserstoff/Sauerstoff-Gemisch kann dabei ohne besondere Vorkehrungen hergestellt werden und mit vergleichsweise langen Verweilzeiten den Mischblock und die vor dem Brennerblock liegenden Teile durchlaufen.

Dies wird dadurch erreicht, daß in dem Brennerblock mindestens ein Sauerstoffkanal angebracht ist, über den weiterer Sauerstoff dem zuvor hergestellten, durch die Kanäle austretenden Kohlenwasserstoff/Sauerstoff-Gemisch aufgegeben wird. Vorzugsweise ist dieser Sauerstoffkanal zentral innerhalb mehrerer Kohlenwasserstoff-Kanäle angebracht. Insbesondere ist dieser Sauerstoffkanal konzentrisch innerhalb mehrerer Kohlenwasserstoff-Kanäle angeordnet. Sind mehrere Sauerstoffkanäle vorhanden, können die beschriebenen Anordnungen beliebig häufig wiederholt werden. Es ist auch möglich, mehrere einzelne Brennerblöcke mit der beschriebenen Sauerstoffkanal/Kohlenwasserstoffkanal-Anordnung zu einem einzigen, zusammenliegenden Brennerblock zusammenzufügen.

Die Zusammensetzung des vor dem Brennerblock hergestellten, nicht zündfähigen Kohlenwasserstoff/Sauerstoff-Gemischs richtet sich natürlich nach der Temperatur, die dieses Gemisch nach dem Vermischen, vor dem Eintritt in den Brennerblock, aufweist. Die Zündgrenzen entsprechender Einsatzgemische sind dem Fachmann bekannt bzw. können entsprechenden Tabellen entnommen oder experimentell bestimmt werden. Es ist bevorzugt, das Kohlenwasserstoff/Sauerstoff-Gemisch bei Raumtemperatur herzustellen und in den Brennerblock eintreten zu lassen. Dabei werden dann bei Einsatz von Erdgas Gemische mit einem Methan-Anteil > 60 Vol.-% verwendet, wenn reiner Sauerstoff verwendet wird.

In einer bevorzugten Ausführungsform der vorliegenden Erfindung ist weiterhin ein Zündbrenner in dem erfindungsgemäßen Brennerblock angebracht. Dieser dient dazu, das sich nach dem Durchtritt durch den Brennerblock bildende Gemisch aus Kohlenwasserstoff und Sauerstoff zu entzünden. Ein solcher Zündbrenner ist prinzipiell nur zum Entzünden des Gemischs zu Beginn der Reaktion notwendig. Gegebenenfalls kann der Zündbrenner auch dazu dienen, eine Zündstabilisierung des brennenden Gemischs sicherzustellen. Der Zündbrenner ist vorzugsweise im Bereich zwischen Kohlenwasserstoff- und Sauerstoffkanälen angebracht.

Der erfindungsgemäße Brennerblock gewährleistet eine Palette an verfahrenstechnischen Vorteilen. So kann das Kohlenwasserstoff/Sauerstoff-Gemisch auf relativ einfache, nur eine grobe Durchmischung der Reaktanden ergebende Art vermischt werden. Außerdem ist es nicht notwendig, die Reaktanden auf höhere Temperaturen vorzuwärmen. Es ist vollkommen ausreichend, die Reaktanden bei Raumtemperatur vorzumischen. Das Durchführen des Mischungsvorgangs bei Raumtemperatur hat außer der günstigen Energiebilanz den Vorteil, daß ein Gemisch hergestellt werden kann, das einen hohen Sauerstoffanteil aufweist, das jedoch noch nicht zündfähig ist und ohne besondere Maßnahmen gehandhabt werden kann. Erst nach dem Durchtritt durch den Brennerkanal, dem Vermischen mit weiterem Sauerstoff sowie dem Erhitzen durch die an dem Brennerblock vorherrschenden Temperaturen entsteht ein zündfähiges Gemisch. Die so entstehenden Gemische sind ideal zur Herstellung von Acetylen/Synthesegas-Gemischen bei Temperaturen < 1400°C. Der Brenner weist bei alldem einen großen Regelbereich nach Durchsatz und Stöchiometrie der Mischung auf.

Der erfindungsgemäße Brennerblock eignet sich zur Herstellung von Acetylen/Synthesegas-Gemischen aus Kohlenwasserstoffen. So können beispielsweise Acetylen/Methanol-Synthesegas, Acetylen/Ammoniak-Synthesegas, Acetylen/Wasserstoff-Reichgas, Acetylen/Kohlenmonoxid-Reichgas, Acetylen/Oxogas und Acetylen/Ethylen-Synthesegas hergestellt werden. Die Reaktion wird bei beliebigem Druck, vorzugsweise Atmosphärendruck, ausgeführt. Als Reaktoren eignen sich vorzugsweise Flammenreaktoren oder Rohrreaktoren auch ohne Ausbildung einer Flammenzone.

Der erfindungsgemäße Brennerblock kann auch bei der Herstellung üblicher Synthesegasgemische eingesetzt werden, bei denen kein Acetylen als Produkt hergestellt wird. Es können hierbei die oben beschriebenen Kohlenwasserstoffe als Ausgangsprodukt verwendet werden. Diese werden mit Sauerstoff in dem gewünschten Verhältnis vermischt und dann dem erfindungsgemäßen Brennerblock aufgegeben. Die Verbrennung wird dann bei den für die Synthesegasherstellung üblichen Bedingungen (Druck, Temperatur, Verweilzeit), bei denen die Acetylen-Ausbeute verschwindet, durchgeführt.

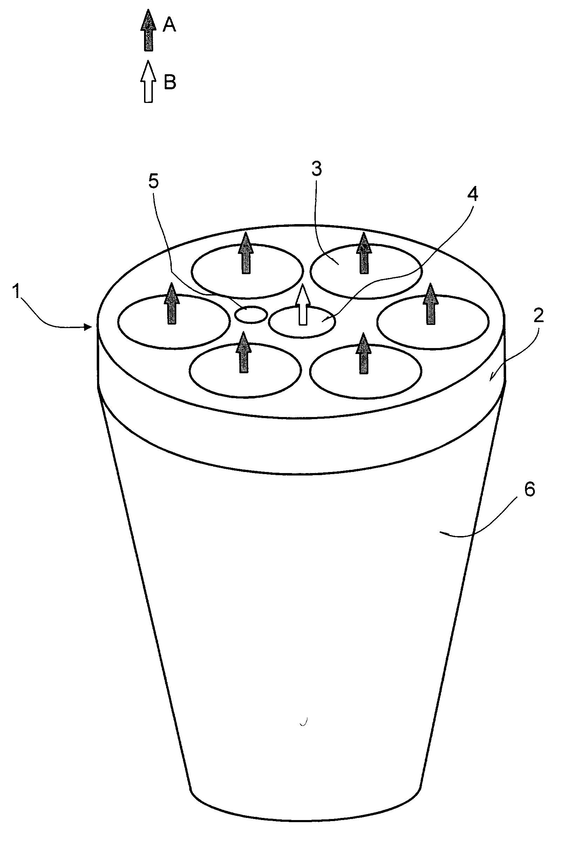

Die beiliegende Fig. 1 zeigt beispielhaft einen erfindungsgemäßen Brennerblock 1 in einer bevorzugten Ausführungsform. Dieser besteht aus einer Brennerbasis 2, in die Kohlenwasserstoff-Kanäle 3 eingelassen sind. Die Kohlenwasserstoff-Kanäle sind konzentrisch um einen Sauerstoffkanal 4 angeordnet. Der zuvor mit Sauerstoff zu einem bei der Mischtemperatur nicht zündfähigen Gemisch vorgemischte Kohlenwasserstoff durchströmt den Brennerblock 1 durch die Kanäle 3 und wird dann nach dem Austritt mit Sauerstoff beaufschlagt. Eine intensive Durchmischung resultiert, danach entzündet sich das Gemisch und verbrennt zu dem gewünschten Gasgemisch bei Temperaturen < 1400°C. Ein Zündbrenner 5 stellt die Zündung des Gemischs sicher. Vorzugsweise geschieht das Mischen des Kohlenwasserstoffs mit dem Sauerstoff in einer dem Brennerblock vorgelagerten Mischkammer 6.

A Kohlenwasserstoff/Sauerstoff-Austritt

B Sauerstoff bzw. Luft/Sauerstoff-Austritt

### Beispiel:

In einem Brennerblock gemäß Fig. 1 werden 200 Nm³/h Erdgas über die Mischkammer mit 90 Nm³/h Sauerstoff gemischt und treten durch die Kohlenwasserstoff-Kanäle aus. Durch den zentralen Sauerstoffkanal strömen 30 Nm³/h Sauerstoff aus. Nach Zündung durch den Zündbrenner stabilisiert sich eine Flamme stromab des Brenners. Das Erdgas-Sauerstoffgemisch wird unter diesen Sauerstoffmangelbedingungen durch die Flamme und die anschließende Nachreaktionszone bei einer mittleren Temperatur von 1300°C in ein Gemisch aus 6 Vol% Acetylen, 7 Vol% Methan, 20 Vol% Kohlenmonoxid, 40 Vol% Wasserstoff, 3 Vol% Kohlendioxid, Rest Wasserdampf sowie kleine Mengen an Ruß und höheren Kohlenwasserstoffen umgesetzt.

## Patentansprüche

1. Brennerblock (1) aufweisend eine Basis (2), mehrere Kohlenwasserstoff-Kanäle (3) zum Durchtritt von als Reaktanden verwendeten Kohlenwasserstoff/Sauerstoff-Gemischen und mindestens einen Sauerstoffkanal (4) zum Durchtritt von Luft, Sauerstoff oder eines Luft/Sauerstoff-Gemischs.

2. Brennerblock nach Anspruch 1, **dadurch gekennzeichnet, daß** der Sauerstoffkanal (4) von mehreren Kohlenwasserstoff-Kanälen (3) umgeben ist.

3. Brennerblock nach Anspruch 1 oder 2, **dadurch gekennzeichnet, daß** der Sauerstoffkanal (4) konzentrisch von Kohlenwasserstoff-Kanälen (3) umgeben ist.

4. Brennerblock nach einem der Ansprüche 1 bis 3, **dadurch gekennzeichnet, daß** ein Zündbrenner (5) vorhanden ist.

5. Brennerblock nach einem der Ansprüche 1 bis 4, **dadurch gekennzeichnet, daß** vor dem Brennerblock (1) eine Mischkammer (6) vorhanden ist.

6. Verwendung eines Brennerblocks nach einem der Ansprüche 1 bis 5 bei der Herstellung von Acetylen/Synthesegas-Gemischen aus Kohlenwasserstoffen und Sauerstoff bei Temperaturen unterhalb 1400°C.

7. Verwendung nach Anspruch 6, **dadurch gekennzeichnet, daß** das Acetylen/Synthesegas-Gemisch ausgewählt ist aus der Gruppe bestehend aus Acetylen/Methanol-Synthesegas, Acetylen/Ammoniak-Synthesegas, Acetylen/Wasserstoff-Reichgas, Acetylen/Kohlenmonoxid-Reichgas, Acetylen/-Oxogas und Acetylen/Ethylen-Synthesegas.

8. Verwendung nach Anspruch 6 oder 7, **dadurch gekennzeichnet, daß** in den Brennerblock ein Kohlenwasserstoff/Sauerstoff-Gemisch eintritt, das bei der dabei herrschenden Temperatur nicht zündfähig ist, vorzugsweise das Kohlenwasserstoff/Sauerstoff-Gemisch bei Raumtemperatur in den Brennerblock eintritt und > 60 Vol.-% Methan enthält.

9. Verwendung nach einem der Ansprüche 6 bis 8, **dadurch gekennzeichnet, daß** als Kohlenwasserstoff Methan, Erdgas, Flüssiggas, Leichtbenzin, Aromatengemische, Pyrolysebenzin oder Rückstandsöl eingesetzt wird.

10. Verwendung eines Brennerblocks nach einem der Ansprüche 1 bis 5 bei der Herstellung von Synthesegas-Gemischen aus Kohlenwasserstoffen und Sauerstoff bei Bedingungen, unter denen die Acetylenausbeute verschwindet.
